# EUROPÄISCHE PATENTSCHRIFT

(11) **EP 1 675 525 B1**
(45) Veröffentlichungstag und Bekanntmachung des Hinweises auf die Patenterteilung: **08.07.2009**
(21) Anmeldenummer: 04790605.2
(22) Anmeldetag: 18.10.2004
(51) Int. Cl.: A61C 19/04, A61N 2/06, A61C 3/00, A61C 19/02

(54) **INTERDENTALSTICK**
INTERDENTAL STICK
INSTRUMENT INTERDENTAIRE

(30) Priorität: 17.10.2003 DE 10348352
(43) Veröffentlichungstag der Anmeldung: 05.07.2006
(73) Patentinhaber: Witte, Achim Hermann, 30655 Hannover (DE)
(72) Erfinder: Witte, Achim Hermann, 30655 Hannover (DE)
(74) Vertreter: Vossius & Partner
(86) Internationale Anmeldenummer: PCT/EP2004/011780
(87) Internationale Veröffentlichungsnummer: WO 2005/034792

(56) Entgegenhaltungen:
- WO-A-01/60279
- DE-U1- 20 013 818
- US-A- 5 676 544
- US-A1- 2002 119 422

## Beschreibung

Die vorliegende Erfindung betrifft ein dentalmedizinisches Handgerät zur Reinigung und Eigenkontrolle für einen Patienten zur Ergebnisbetrachtung seiner Zahnpflegebemühungen welches insbesondere einen Magneten für zusätzliche magnetotherapeutische Anwendungen aufweist.

Die im zahnärztlichen Gebrauch befindlichen herkömmlichen Geräte sind nur rein auf den professionellen Praxisbedarf zugeschnitten. Diese Geräte bestehen üblicherweise aus Edelstahl und weisen Spitzen und traumatisierende Arbeitsenden auf. Die DE 19913 544 offenbart ein dentalmedizinisches Handgerät mit zwei Arbeitsenden, welches den Stand der Technik ausführlich diskutiert um ein verbessertes dentalmedizinisches Handgerät insbesondere zur Eigenbehandlung eines Patienten zur Verfügung zu stellen.

Ein anderes Gebiet zur Vorbeugung gegen Körperliche Beschwerden beschäftig sich mit magnetotherapeutischen Anordnungen die beispielsweise als Bettauflage (DE 41 36 374) oder als Halsketten oder Armbänder (DE 198 07 464) mit integrierten Permanentmagneten angeboten werden, und aufgrund ihrer Magnetfeldlinien zur positiven Stimulation des Organismus getragen werden. All diesen Anordnungen gemeinsam, ist das Aufweisen von Permanentmagneten, die vorzugsweise in engem Kontakt zum Körper angebracht werden, damit die Magnetfeldlinien in das Körpereigene Gewebe des Trägers eindringen können. Für weiter Diskussionen der Wechselwirkung des menschlichen Organismus mit magnetischen Feldern wird auf die Fachliteratur verwiesen.

Dokument US 5 676 544 offenbart ein dentalmedizinisches Handgerät zur Diagnose von Zahntaschentiefen, welches einstückig ist.

Der vorliegenden Erfindung liegt nun die Aufgabe zugrunde ein Multifunktions-Gerät bereitzustellen das neben der dentalmedizinischen Gesundheitsvorsorge eine magnetotherapeutische Anordnung aufweist. Eine Person die das Gerät bei sich trägt ist dem magnetischen Feld eines Permanentmagneten ausgesetzt der das Wohlbefinden der Person positiv stimulieren kann.

Eine weitere Aufgabe der Erfindung besteht darin, ein einfach zu handhabendes dentalmedizinisches Handgerät, auch as Interdentalstick bezeichnet, bereitzustellen welches sich insbesondere zur Eigenbehandlung und Eigenmessung der Taschentiefen eignet.

Weiterhin besteht die Aufgabe der Erfindung darin, ein leicht zu handhabendes und sicher zu greifendes Handgerät mit verbessertem Griffelement bereitzustellen.

Eine weitere Aufgabe der Erfindung besteht darin, eine Box oder Schatulle zum sicheren Aufbewahren und Transportieren des Handgeräts bereitzustellen.

Diese Aufgabe wird mit den Merkmalen der Patentansprüche gelöst.

Dabei geht die Erfindung einerseits von dem Grundgedanken aus, ein dentalmedizinisches Handgerät mit einem Arbeitsende im Bereich einer Spitze mit doppelt abgewinkelten Ende, zur Entfernung von Speiseresten und Plaque aus Zahnzwischenräumen und zur Diagnose von Zahntaschentiefen auszubilden. Insbesondere ist es bevorzugt, das Arbeitsende des erfindungsgemäßen Handgeräts aus Kunststoff, wie z.B. Polycarbonat, auszubilden und das Arbeitsende mit makro- und/oder mikroskopischen Rundungen zu versehen. Darüber hinaus ist es vorteilhaft, das Arbeitsende zur antimikrobiellen Reinigung mit tiefengerauhten Oberflächen zu versehen. Zur Diagnose der Zahntaschentiefen weist das Arbeitsende vorzugsweise eine Skalierung und/oder Farbeinteilung auf. Weiterhin wird das Handgerät einstückig gefertigt, was ein kostengünstiges Herstellungsverfahren ermöglicht. Als Grundmaterial eignet sich hierfür Metall oder Kunststoff. Kunststoff lässt sich einerseits sehr einfach verarbeiten und kann nahezu in beliebigen Härtegraden hergestellt werden. So kann es durchaus wünschenswert sein, einen Kunststoff zu verwenden, der aufgrund seiner geringen Härte, das Verletzungsrisiko an Zahn und Zahnfleisch minimiert. Zur sicheren Handhabung wird der Griff des Handgerätes vorzugsweise Kunststoff oder einem gummiähnlichen Kunststoff überzogen bzw. beschichtet um ein versehentliches Abrutschen während des Gebrauchs zu vermeiden. Noch bevorzugter wird Gummi für die Beschichtung des Griffbereichs verwendet.

Der erfindungsgemäße Interdentalstick ist ein Multifunktionsgerät, das einerseits zur Reinigung und/oder Kontrolle der Zahnzwischenräume bzw. Zahntaschen dient und andererseits von dem Grundgedanken ausgeht, eine Magnetotherapeutische Anordnung bereitzustellen. Insbesondere, wenn das Handgerät am Körper getragen wird, dringt das Magnetfeld des Permanentmagneten, der im Schaft des Interdentalsticks integrierten ist, in den Körper des Trägers ein und ruft eine Wechselwirkung mit dem Körper hervor, die in gewissem Umfang wünschenswert ist und den Träger des Gerätes positiv beeinflussen kann.

Das erfindun gsgemäße dentalmedizinische Handgerät hat gegenüber bekannten Geräten neben einer sicheren Handhabung durch einen Antirutschgriff und magnetotherapeutische Wirkung weitere mechanische Vorteile insbesondere zur Zahnpflege bzw. -Kontrolle, da es durch die Materialwahl, d.h. deren Härte und Oberflächenbeschaffenheit atraumatisch ist. Der Interdentalstick eignet sich insbesondere zum Entfernen von Speiseresten (Detritus) und Plaque aus den Zahnzwischenräumen in Kooperation mit Zahnseide. Schwer zugängliche Stellen in den Zahnzwischenräumen, die nur schwer mit der Zahnbürste oder Zahnseide zugänglich sind, können aufgrund der speziellen Form des Arbeitsendes des Interdentalsticks gezielt gereinigt werden. Aufgrund einfach herstellbarer mikroskopischer und makroskopischer Rundungen besteht eine geringe Autoläsionsgefahr für den Patienten. Darüber hinaus ist das erfindungsgemäße Handgerät flexibel wodurch während der Reinigung durch einen Patienten keine überproportionalen Kräfte auftreten können, die Schäden im Dentalbereich verursachen könnten. Darüber hinaus besteht bei Verwendung von Kunststoffen an dem Arbeitsende, wie z.B. Polycarbonat, die Möglichkeit, die Oberflächen derart zu beschaffen, daß im mikroskopischen, makroskopischen bzw. ultrastrukturellen Bereich eine Rauhigkeit vorhanden ist, die zusätzlich zur Grobreinigung eine gewisse Feinreinigung ermöglicht, so daß Plaque und Bakterien während eines Reinigungsvorgangs hängenbleiben und somit das erfindungsgemäße Handgerät antimikrobiell wirkt. Die Rauhigkeit der Obertflächen bietet zudem die Möglichkeit, Desinfektionslösungen die aufgrund von Adhäsionskräften an der Oberfläche haften, gezielt in die Zahnzwischenräume zu bringen.

Ferner ist das erfindungsgemäße dentalmedizinische Handgerät gut sterilisierbar und leicht, so daß eine mühelose und leichte Handhabung ermöglicht ist. Die an dem Arbeitsende vorsehbare Skalierung bzw. Farbeinteilung ermöglicht einem ungeübten Benutzer auf einfache Weise, eine Selbstdiagnose zu erstellen und damit das Ergebnis seiner Zahnpflegebemühungen zu verifizieren. Darüber hinaus ist ein wesentlicher Vorteil des erfindungsgemäßen dentalmedizinischen Handgeräts dadurch gegeben, daß es als Multifunktionsgerät ausbildbar ist, wobei die magnetischen Feldlinien beispielsweise beim Tragen des Geräts in einer körpernahen Tasche in das Gewebe des Trägers eindringen und als magnetotherapeutisches Gerät eingesetzt wird.

Weiterhin wird das Handgerät bevorzugt in einer erfindungsgemäßen Aufbewahrungsbox aufbewahrt, die einerseits dazu dient, das spitze Arbeitsendes beim Transport zu schützen und andererseits kann die Aufbewahrungsbox dazu dienen, die Ausbreitung des Magnetfeldes mit Abschirmelementen innerhalb der Box gezielt zu richten. Im Inneren der Aufbewahrungsbox kann zusätzlich eine Kurzanleitung zur Anwendung des Interdentalsticks aufgedruckt oder beigelegt werden.

Das erfindungsgemäße dentalmedizinische Handgerät wird nachstehend anhand von bevorzugten Ausführungsformen unter Bezugnahme auf die Zeichnungen beispielhaft beschrieben. Es zeigen:
- Figur 1: eine schematische Seitenansicht eines dentalmedizinischen Handgeräts mit Gummigriff;
- Figur 2: eine Vergrößerung des in Figur 1 mit II bezeichnenden Ausschnitts des Arbeitsendes;
- Figur 3: eine schematische Seitenansicht einer Ausführungsform des erfindungsgemäßen dentalmedizinischen Handgeräts mit integriertem Permanentmagneten; und
- Figur 4: eine schematische Darstellung einer Aufbewahrungsbox.

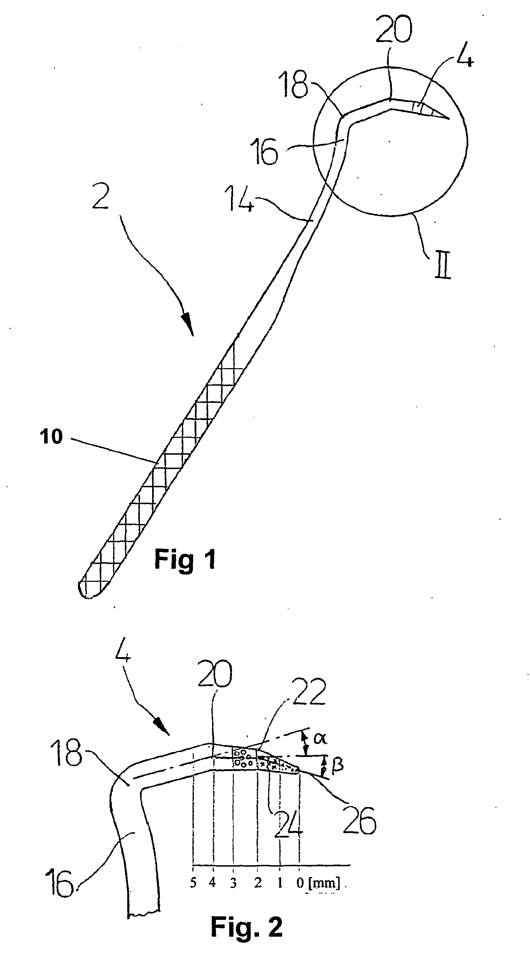

Das in Figur 1 dargestellte dentalmedizinische Handgerät 2 hat im wesentlichen ein Arbeitsende 4. Das Arbeitsende 4 ist in Figur 2 vergrößert dargestellt. Das Arbeitsende 4 ist mit einem Griffabschnitt 8 verbunden.

Vorzugsweise ist das Arbeitsende 4 mit den Griffabschnitten 8 einteilig ausgebildet.

Der Griffabschnitt 8 ist vorzugsweise mit einem Gummibelag 10 oder einem Gummiähnlichen weichen Kunststoff beschichtet um das Handgerät rutschsicher zu handhaben. Vorzugsweise weist der Gummibelag 10 Rillen und/oder Vertiefungen und/oder Erhöhungen auf um eine zusätzliche Antirutschwirkung zu erreichen.

Das dentalmedizinische Handgerät 2 weist vorzugsweise mindestens ein aus Kunststoff hergestelltes Arbeitsende 4 auf, kann jedoch auch vollständig aus Kunststoff gefertigt sein. Bevorzugt wird als Kunststoff insbesondere Polycarbonat und/oder Polyamid. Dadurch kann insbesondere eine gute Atraumatizität, Flexibilität, antimikrobielle Wirkung, Sterilisierbarkeit, Hygiene und einfache Handhabbarkeit realisiert werden.

Das Arbeitsende 4 ist, wie in Fig. 1 dargestellt, mit seinem Griffabschnitt 8 über ein dazwischenliegendes, gebogenes Verbindungselement 14 verbunden. Vorzugsweise ist dieses Verbindungselement 14 in einem sich vom Griffabschnitt 8 weg erstreckenden Bereich 16 leicht nach oben gebogen und anschließend mittels einer weiteren Biegung bzw. einem Knick 18 beispielsweise um einen Winkel γ nach unten gebogen. Der Winkel γ liegt vorzugsweise zwischen 80° und 150° und besonders bevorzugt zwischen 90° und 135°. Daran anschließend ist das eigentliche erste Arbeitsende 4 mit einer doppelten Abwinkelung 20 bzw. 22 im Bereich einer Spitze 26 vorgesehen. Die doppelt abgewinkelte Form des ersten Arbeitsendes 4 ermöglicht einen geringen Hebelarm, so daß ein Patient sich nicht selbst verletzen kann, und erlaubt dem Benutzer auch, ungünstig gewinkelte Nischen zu reinigen. Die Winkel α und β der ersten bzw. zweiten Abwinkelung 20 und 22 liegen vorzugsweise jeweils zwischen 15° und 25°, stärker bevorzugt zwischen 20° und 30° und am stärksten bevorzugt bei etwa 25°. Die Summe der Winkel α und β ist vorzugsweise etwa 50°, wobei die Winkel α und β gleich oder unterschiedlich groß ausgebildet sein können. Mit dieser Form des ersten Arbeitsendes 4 bzw. der Spitze 26 lassen sich besonders vorteilhafte "Schaufeleffekte", "Hebeleffekte" und Krümmungskongruenzen erzielen.

Das Arbeitsende 4 weist vorzugsweise eine Skalierung und/oder Farbeinteilung 24 auf, mit der die Zahntaschentiefe auf einfache Weise kontrolliert werden kann. Die Skalierung 24 ist beispielsweise in einen gesunden Bereich, einen Übergangsbereich und einen kranken Bereich eingeteilt. Dazu weist die Skalierung 24 beispielsweise einen von einer Spitze 26 ca. 1 mm entfernten ersten Teilstrich, einen ca. 2 mm davon entfernten zweiten Teilstrich und einen etwa 3 mm davon entfernten dritten Teilstrich auf. Kann das erste Arbeitsende 4 bis zum ersten Teilstrich in die Zahntasche eines Patienten eingeführt werden, so ist dieser Bereich gesund. Kann das Gerät bis zum zweiten Teilstrich eingeführt werden, so ist dieser Bereich in einem Übergangsstadium zwischen gesund und krank. Falls das erste Arbeitsende 4 bis zum dritten Teilstrich oder darüber hinaus in die Zahntasche einführbar ist, ist eine Zahntaschenbehandlung erforderlich. Zur leichteren Erkennbarkeit der Eintauchtiefe können die einzelnen Abschnitte bzw. Teilstriche der Skalierung 24 eingefärbt sein, wie dies in Figur 2 anhand der in den drei Bereichen verwendeten Symbole dargestellt ist.

Das am dentalmedizinischen Handgerät 2 ausgebildete Arbeitsende ist vorzugsweise abgerundet, so daß Verletzungen vermieden werden. Darüber hinaus ist insbesondere das Arbeitsende 4 oder aber das gesamte Handgerät 2 flexibel ausgebildet, um nur eine vorherbestimmte definierte Kraft mit dem Gerät aufbringen zu können. Die Oberfläche der Arbeitsende 4 ist vorzugsweise aufgerauht, so daß Grob- und Feinreinigungen mit dem erfindungsgemäßen Gerät durchgeführt werden können. Zudem können Desinfektionslösungen oder sonstige Flüssigkeiten, die aufgrund von Adhäsionskräften an der rauen Oberfläche des Arbeitsendes haften, gezielt in die Zahnzwischenräume gebracht werden.

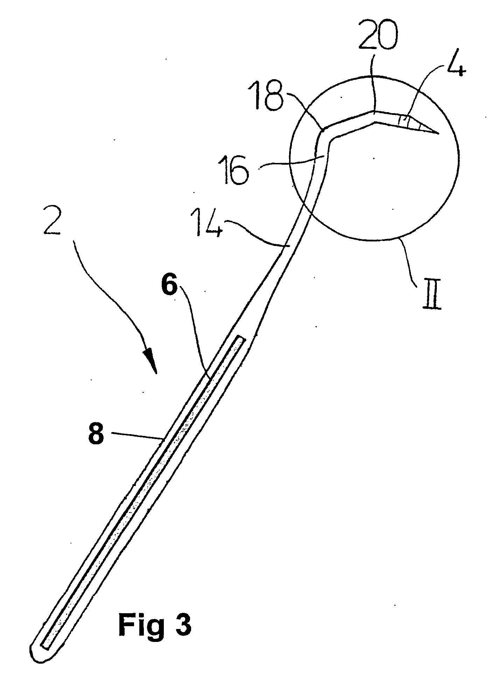

Die erfindungsgemäße Ausführungsform, die in Figur 3 dargestellt ist, hat im wesentlichen dieselbe Form wie die in Figur 1 dargestellte Ausführungsform, weist jedoch im Griffbereich integriert einen Permanentmagneten 6 auf. Der Permanentmagnet ist hierbei vorzugsweise innerhalb des Griffelements 8 axialsymmetrisch angebracht.

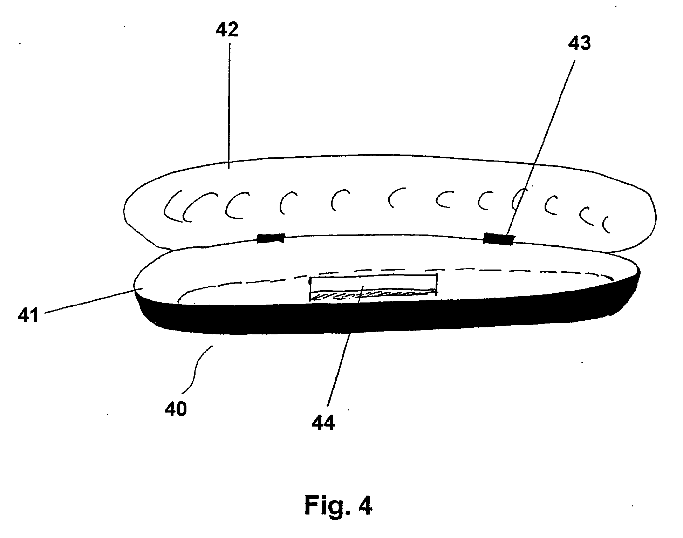

Um das Handgerät auch unterwegs auf oder Reisen sicher zu transportieren ohne sich selbst an dem Arbeitsende 4 zu verletzen, wird das Handgerät vorzugsweise in einer Transportschatulle oder Aufbewahrungsbox 40, die in Figur 4 dargestellt ist, aufbewahrt. Die Aufbewahrungsbox besteht im wesentlichen aus je einem länglichen Unterteil 41 und einem Oberteil 42 wobei mindestens eines der beiden Teil als Schale ausgebildet ist und die beiden Teile zusammengesetzt, einen Hohlraum zum Aufbewahren des Handgeräts bilden. Die beiden Teile sind vorzugsweise an einer Längsseite mit mindestens einem Scharnier 43 drehbar miteinander verbunden, um die Aufbewahrungsbox mittels einer Klappbewegung zu Öffnen bzw. zu Schließen. Das Ober- und/oder Unterteil bestehen aus einem Material, welches die magnetischen Feldlinien des Permanentmagneten, der im Griff des aufbewahrten Haltegeräts angebracht ist nicht abschirmen. In einer anderen Ausführungsform kann es jedoch wünschenswert sein, dass mindestens ein Teil der Aufbewahrungsbox beispielsweise aus Metall oder aus einem anderen Material besteht welches die magnetischen Feldlinien in einer bestimmten Richtung abschirmt um so beispielsweise die Umwelt oder empfindliche elektrische Geräte keinen magnetischen Feldlinien auszusetzen. Weiterhin weist die Aufbewahrungsbox 40 einen Halteclip 44 auf, der den Interdentalstick im Inneren der Box sichert.

## Patentansprüche

1. Einstückiges dentalmedizinisches Handgerät (2) mit einem im Bereich einer Spitze (26) doppelt abgewinkelten Arbeitsende (4) zur Diagnose von Zahntaschentiefen und zur Reinigung von Zähnen und/oder Zahnzwischenräumen,
**dadurch gekennzeichnet, dass**
das dentalmedizinische Handgerät zumindest im Bereich des Griffabschnitts (8) einen integrierten Permanentmagneten (6) aufweist.

2. Handgerät nach Anspruch 1, wobei das Handgerät außen im Bereich des Griffabschnitts (8) eine Griffummantelung (10) aufweist.

3. Handgerät nach Anspruch 2, wobei die Griffummantelung (10) aus weichem Kunststoff oder Gummi besteht.

4. Handgerät nach einem der Ansprüche 1 bis 3 wobei das Arbeitsende (4) aus Kunststoff gebildet ist.

5. Handgerät nach Anspruch 4, wobei der Kunststoff aus Polycarbonat ist.

6. Handgerät nach einem der Ansprüche 1 bis 5, wobei das Arbeitsende (4) makro- und/oder mikroskopische Rundungen aufweist.

7. Handgerät nach einem der Ansprüche 1 bis 6, wobei das Arbeitsenden (4) flexibel ausgebildet ist.

8. Handgerät nach einem der Ansprüche 1 bis 7, wobei das Arbeitsende (4) tiefengerauhte Oberflächen zur antimikrobiellen Reinigung der Zähne und/oder Zahnzwischenräume aufweist.

9. Handgerät nach einem der Ansprüche 1 bis 8, wobei das Arbeitsende (4) an einem Endabschnitt (22) eine Skalierung und/oder Farbeinteilung (24) zur Diagnose der Zahntaschentiefe aufweist.

10. Handgerät nach Anspruch 9, wobei die Skalierung und/oder Farbeinteilung (24) von einer Spitze (26) des Arbeitsendes (4) gemessen bis 2 mm eine erste, bis 3 mm eine zweite und bis 4 mm eine dritte Teilung aufweist.

11. Handgerät nach einem der Ansprüche 1 bis 10, wobei die doppelte Abwinklung einen ersten Winkel α zwischen 15° und 35°, bevorzugt zwischen 20° und 30° und besonders bevorzugt von etwa 25°, sowie einen zweiten Winkel β zwischen 15° und 35°, bevorzugt zwischen 20° und 30° und besonders bevorzugt von etwa 25°aufweist.

12. Handgerät nach Anspruch 11, wobei die Summe der Winkel α und β etwa 50° beträgt.

13. Kombination des dentalmedizinischen Handgeräts nach einem der Ansprüche 1 bis 12 mit einer Aufbewahrungsbox (40) zum Aufbewahren des Handgeräts (2), wobei die Aufbewahrungsbox (40) aus einem länglichen Unterteil (41) und Oberteil (42) besteht, die beiden länglichen Teile zusammengesetzt einen Hohlraum zum Aufbewahren bilden, und das Oberteil (42) mit dem Unterteil (41) über mindestens ein Scharnier (43) verbunden ist
**dadurch gekennzeichnet, dass**
das Unterteil (41) im Inneren eine Halteeinrichtung (44) zum Halten des Handgeräts aufweist.

14. Kombination nach Anspruch 13, wobei die Halteeinrichtung ein Clip ist.

15. Kombination nach Anspruch 13, wobei die Halteeinrichtung aus Metall besteht und durch magnetischen Kontakt zum Handgerät gehalten wird.

## Claims

1. An integrally formed dental medical hand tool (2) comprising a working end (4) that is double-angled in the area of a tip (26) for the diagnosis of the depth of periodontal pockets and for cleaning teeth and/or interdental gaps,
**characterized in that**
the dental medical hand tool comprises at least in the area of the handle portion (8) an integrated permanent magnet (6).

2. The hand tool according to claim 1, wherein the hand tool comprises on its outside a handle shell (10) in the area of the handle portion (8).

3. The hand tool according to claim 2, wherein the handle shell (10) is made of soft plastic material or rubber.

4. The hand tool according to any one of claims 1 to 3, wherein the working end (4) is made of plastic material.

5. The hand tool according to claim 4, wherein the plastic material is polycarbonate.

6. The hand tool according to any one of claims 1 to 5, wherein the working end (4) comprises macroscopic and/or microscopic curves.

7. The hand tool according to any one of claims 1 to 6, wherein the working end (4) is flexible.

8. The hand tool according to any one of claims 1 to 7, wherein the working end (4) comprises depth-roughened surfaces for antimicrobially cleaning the teeth and/or interdental gaps.

9. The hand tool according to any one of claims 1 to 8, wherein one end portion (22) of the working end (4) comprises a scaling and/or color gradation (24) for the diagnosis of the depth of periodontal pockets.

10. The hand tool according to claim 9, wherein the scaling and/or color gradation (24) comprises, when measured from a tip (26) of the working end (4), a first division up to 2 mm, a second division up to 3 mm and a third division up to 4 mm.

11. The hand tool according to any one of claims 1 to 10, wherein the double-angling comprises a first angle α between 15° and 35°, preferably between 20° and 30° and particularly preferably of about 25°, as well as a second angle β between 15° and 35°, preferably between 20° and 30° and particularly preferably of about 25°.

12. The hand tool according to claim 11, wherein the sum of the angles α and β is about 50°.

13. A combination of the dental medical hand tool according to any one of claims 1 to 12 with a storage box (40) for storing the hand tool (2), wherein the storage box (40) consists of an elongate lower part (41) and upper part (42), the two elongate parts form a storage space when combined, and the upper part (42) is connected with the lower part (41) by means of at least one hinge (43),
**characterized in that**
the lower part (41) comprises in its interior a holding means (44) for holding the hand tool.

14. The combination according to claim 13, wherein the holding means is a clip.

15. The combination according to claim 13, wherein the holding means is made of metal and held by magnetic contact with the hand tool.

## Revendications

1. Instrument dentaire monobloc (2) doté d'une extrémité active (4) recourbée deux fois dans la zone d'une pointe (26) pour le diagnostic de crevasses gingivales et pour le nettoyage de dents et/ou d'interstices, **caractérisé en ce que** l'instrument dentaire présente au moins dans la zone du manche (8) un aimant permanent intégré (6).

2. Instrument selon la revendication 1, sachant que l'instrument présente à l'extérieur dans la zone du manche (8) une enveloppe de manche (10).

3. Instrument selon la revendication 2, sachant que l'enveloppe de manche (10) se compose de plastique ou de caoutchouc mou.

4. Instrument selon l'une quelconque des revendications 1 à 3, sachant que l'extrémité active (4) est constituée de matière plastique.

5. Instrument selon la revendication 4, sachant que la matière plastique est en polycarbonate.

6. Instrument selon l'une quelconque des revendications 1 à 5, sachant que l'extrémité active (4) présente des arrondis macroscopiques et/ou microscopiques.

7. Instrument selon l'une quelconque des revendications 1 à 6, sachant que l'extrémité active (4) est conçue de manière flexible.

8. Instrument selon l'une quelconque des revendications 1 à 7, sachant que l'extrémité active (4) présente des surfaces rugueuses en profondeur pour le nettoyage antimicrobien des dents et/ou des interstices.

9. Instrument selon l'une quelconque des revendications 1 à 8, sachant que l'extrémité active (4) présente à un tronçon terminal (22) une graduation et/ou une échelle de couleur (24) pour diagnostiquer la crevasse gingivale.

10. Instrument selon la revendication 9, sachant que la graduation et/ou l'échelle de couleur (24) présente une première graduation mesurée de la pointe (26) de l'extrémité active (4) jusqu'à 2 mm, une deuxième graduation jusqu'à 3 mm et une troisième graduation jusqu' à 4 mm.

11. Instrument selon l'une quelconque des revendications 1 à 10, sachant que la double courbure présente un premier angle α compris entre 15° et 35°, de préférence entre 20° et 30° et mieux encore d'environ 25° ainsi qu'un second angle β compris entre 15° et 35°, de préférence entre 20° et 30° et mieux encore d'environ 25°.

12. Instrument selon la revendication 11, sachant que la somme des angles α et β s'élève à environ 50°.

13. Combinaison de l'instrument dentaire selon l'une quelconque des revendications 1 à 12 avec un étui (40) pour ranger l'instrument (2), sachant que l'étui (40) se compose d'une partie inférieure (41) et d'une partie supérieure (42) longitudinales, les deux parties longitudinales assemblées formant un espace creux de logement, et la partie supérieure (42) étant reliée à la partie inférieure (41) par l'intermédiaire d'au moins une charnière (43),
**caractérisée en ce**
**que** la partie inférieure (41) présente à l'intérieur un dispositif de maintien (44) pour maintenir l'instrument.

14. Combinaison selon la revendication 13, sachant que le dispositif de maintien est un clip.

15. Combinaison selon la revendication 13, sachant que le dispositif de maintien est composé de métal et est maintenu par contact magnétique à l'instrument.
